(19) Europäisches Patentamt
European Patent Office
Office européen des brevets

(11) Veröffentlichungsnummer: **0 388 789 A1**

# (12) EUROPÄISCHE PATENTANMELDUNG

(21) Anmeldenummer: **90104890.0**

(22) Anmeldetag: **15.03.90**

(51) Int. Cl.$^5$: **C07D 495/14, A61K 31/55**

(30) Priorität: **18.03.89 DE 3909012**

(43) Veröffentlichungstag der Anmeldung:
**26.09.90 Patentblatt 90/39**

(84) Benannte Vertragsstaaten:
**AT BE CH DE DK ES FR GB GR IT LI LU NL SE**

(71) Anmelder: **BOEHRINGER INGELHEIM KG**
**Postfach 200**
**D-6507 Ingelheim am Rhein(DE)**
(84) **BE CH DE DK ES FR GR IT LI LU NL SE AT**

Anmelder: **BOEHRINGER INGELHEIM INTERNATIONAL GmbH**
**Postfach 20**
**D-6507 Ingelheim am Rhein(DE)**
(84) **GB**

(72) Erfinder: **Stransky, Werner, Dr.**
**Im Hippel 24**
**D-6535 Gau-Algesheim(DE)**
Erfinder: **Langbein, Adolf, Dr.**
**Am Goldberg 6**
**D-6535 Gau-Algesheim(DE)**
Erfinder: **Brandt, Klaus, Dr.**
**Georg-Scheuing-Strasse 22**
**D-6507 Ingelheim am Rhein(DE)**
Erfinder: **Weber, Karl-Heinz, Dr.**
**Kaiser-Karl-Strasse 11**
**D-6535 Gau-Algesheim(DE)**
Erfinder: **Walther, Gerhard, Dr.**

**Verstorben(DE)**

## (54) Verbessertes Verfahren zur Herstellung von enantiomeren Hetrazepinen.

(57) Die Erfindung betrifft ein verbessertes Verfahren zur Herstellung enantiomerenreiner Hetrazepine durch eine stereospezifische Synthese ausgehend von Cyclopentan-3-carbonsäureestern.

EP 0 388 789 A1

## Verbessertes Verfahren zur Herstellung von enantiomerenreinen Hetrazepinen

Die Erfindung betrifft ein verbessertes Verfahren zur Herstellung von enantiomerenreinen substituierten Hetrazepinen. Von besonderem Interesse sind enantiomerenreine Hetrazepine der allgemeinen Formel

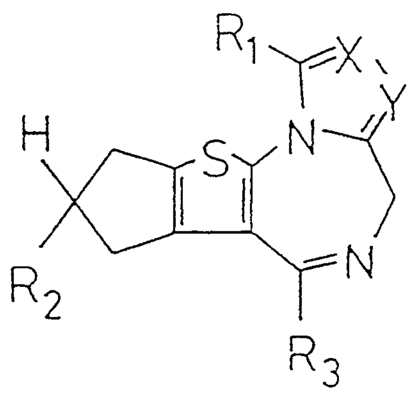

worin

$R_1$ Wasserstoff, eine verzweigte oder unverzweigte Alkylgruppe mit 1 bis 4 Kohlenstoffatomen, bevorzugt Methyl, die gegebenenfalls durch Hydroxy oder Halogen substituiert sein kann, eine Cyclopropylgruppe, eine Cyclobutylgruppe, Cyclopentylgruppe, Halogen, bevorzugt Chlor und Brom;

$R_2$ COOR ($R = C_1$-$C_4$-Alkyl), COOH oder eine Säureamidgruppe;

$R_3$ Phenyl, wobei der Phenylring ein oder mehrfach, bevorzugt in 2-Stellung, durch Methyl, bevorzugt Halogen, Nitro und/oder Trifluormethyl substituiert sein kann;

$R_3$ Pyridyl, substituiertes Pyridyl, Thienyl oder substituiertes Thienyl, wobei der Heterocyclus bevorzugt über die 2-Stellung verknüpft ist;

und

X und Y beide Stickstoff oder X CH und Y Stickstoff bedeuten können.

Die obengenannten Hetrazepine sind beispielsweise aus der europäischen Patentanmeldung 254 245 als Arzneimittel mit PAF-antagonistischer Wirkung bekannt. Zur Definition der Säureamidgruppe wird inhaltlich ebenfalls auf die europäische Patentanmeldung 254 245 Bezug genommen.

Als Säureamidgruppe gemäß dieser Definition ist auch ein Rest der allgemeinen Formel

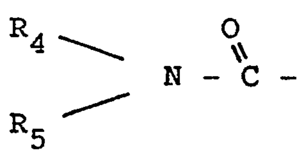

zu verstehen,

worin

$R_4$ und $R_5$, die gleich oder verschieden sein können, Wasserstoff, Phenyl, substituiertes Phenyl, eine gegebenenfalls substituierte Cycloalkylgruppe mit 3 bis 6 Kohlenstoffatomen, eine verzweigte oder unverzweigte Alkyl-, Alkenyl-oder Alkinylgruppe mit 1 bis 10 - bevorzugt 1 - 4 - Kohlenstoffatomen, die gegebenenfalls durch {Halogen, Hydroxy, Nitro, Phenyl, substituiertes Phenyl, Amino, substituiertes Amino, Alkoxy 1 - 8, bevorzugt 1 - 4} substituiert sein kann;

$R_4$ und $R_5$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoff gebundener 5-, 6- oder 7-gliedriger heterocyclischer Ring;

oder

$R_4$ und $R_5$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann,

wobei jedes weitere Stickstoffatom durch eine verzweigte oder unverzweigte Alkylgruppe mit 1 bis 4 Kohlenstoffatomen, bevorzugt Methyl, substituiert sein kann.

Als Alkylgruppen (auch soweit sie Bestandteil anderer Reste sind) werden beispielsweise genannt: Methyl, Ethyl, Propyl, iso-Propyl, Butyl, iso-Butyl, sec. Butyl, tert-Butyl, Pentyl, iso-Pentyl, Hexyl, Heptyl, Octyl, Nonyl und Dekanyl.

Als Alkenylgruppen werden beispielsweise oben genannte Alkylgruppen bezeichnet soweit sie mindestens eine Doppelbindung aufweisen, wie zum Beispiel Vinyl (soweit keine unbeständigen Enamine gebildet werden), Propenyl, iso-Propenyl, Butenyl, Pentenyl, Hexenyl.

Als Alkinylgruppen werden beispielsweise oben genannte Alkylgruppen bezeichnet, soweit sie mindestens eine Dreifachbindung aufweisen, wie zum Beispiel Propargyl, Butinyl, Pentinyl, Hexinyl.

Als Cycloalkylreste mit 3 - 6 Kohlenstoffatomen werden beispielsweise Cyclopropyl, Cyclobutyl, Cyclopentyl oder Cyclohexyl bezeichnet, die durch verzweigtes oder unverzweigtes Alkyl mit 1 bis 4 Kohlenstoffatomen, Hydroxy, und/oder Halogen substituiert sein können.

Beispielhaft für substituiertes Phenyl werden genannt:

3-Chlorphenyl, 4-Chlorphenyl, 3-Bromphenyl, 4-Bromphenyl, 4-Fluormethyl, 2-Chlorphenyl, 2-Bromphenyl, 3-Fluorphenyl, 2,3-Dichlorphenyl, 2-Methylphenyl, 4-Methylphenyl, 3-Ethylphenyl, 4-Propylphenyl, 4-Isopropylphenyl, 4-Butylphenyl, 4-tert-Butylphenyl, 4-Iso-butylphenyl, 4-Pentylphenyl, 2,4-Dimethylphenyl, 2-Trifluormethylphenyl, 3-Trifluormethylphenyl, 4-Trifluormethylphenyl, 2-Methoxyphenyl, 4-Methoxyphenyl, 3-Ethoxyphenyl, 2-Propoxyphenyl, 4-Butoxyphenyl, 2,4-Dimethoxyphenyl, 3,4,5-Trimethoxyphenyl, 2-Chlorbenzyl, 2,3-Dichlorbenzyl, 2-Methylbenzyl, 2-Trifluormethylbenzyl, 4-Methoxybenzyl, 3,4,5-Trimethoxybenzyl, 2-(2-Chlorphenyl)ethyl,

Beispiele für gegebenenfalls substituierte gesättigte oder ungesättigte heterocyclische 5-, 6- oder 7-gliedrige Ringe bzw. Heteroarylreste sind:

Pyrrol, Pyrrolin, Pyrrolidin, 2-Methylpyrrolidin, 3-Methylpyrrolidin, Piperidin - gegebenenfalls durch $C_1$-$C_4$ Alkyl ein- oder mehrfach substituiert -Piperazin, N-Methylpiperazin, N-Ethylpiperazin, N-n-Propylpiperazin, N-Benzylpiperazin, Morpholin, Thiomorpholin, Imidazol, Imidazolin, Imidazolidin, Triazol, Pyrazol, Pyrazolin, Pyrazolidin, Triazin, 1, 2, 3, 4 - Tetrazin, 1, 2, 3, 5 - Tetrazin, 1, 2, 4, 5 - Tetrazin - wobei die genannten Heterocyclen durch Alkyl mit 1 bis 4 Kohlenstoffatomen - bevorzugt Methyl - substituiert sein können;

Als heterocyclische Reste, die über ein Kohlenstoffatom verknüpft sein können, werden beispielsweise Thiophen, 2-Methylthiophen, Furan, Tetrahydrofuran, 2-Methyltetrahydrofuran, Tetrahydrofuran, 2-Hydroxymethylfuran, α-Pyran, γ-Pyran, 1,3-Dioxolan, 1,2-Oxathiolan, 1,2-Oxathiepan, Tetrahydro-pyran, Thiolan, 1,3-Dithian, 1,3-Dithiolan, 1,3-Dithiolen, genannt, wobei der Heterocyclus durch $C_1$-$C_4$ Alkyl, $C_1$-$C_4$ Alkoxy, Halogen substituiert sein kann.

Als Heterocyclus im Rahmen der zuvor angegebenen Definition steht im allgemeinen für einen 5- bis 6-gliedrigen Ring, der als Heteroatome Sauerstoff, Schwefel und/oder Stickstoff enthalten kann, wie zum Beispiel Thienyl, Furyl, Pyridyl, Pyrimidyl, Pyrazinyl, Pyrazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl und Indolyl genannt.

Der Heterocyclus kann durch Halogen, Hydroxy und/oder verzweigtes oder unverzweigtes Alkyl mit 1 bis 4 Kohlenstoffatomen verzweigtes oder unverzweigtes Alkoxy mit 1 bis 4 Kohlenstoffatomen substituiert sein.

In der europäischen Patentanmeldung 254 245 wird die Herstellung der Racemate sowie die säulenchromatographische Trennung der Enantiomeren mit Hilfe eines optisch aktiven Trägermaterials offenbart. Ein wesentlicher Nachteil dieses Verfahrens ist, daß die Trennung erst auf der Stufe des Endprodukts erfolgt und dies nur unter Zuhilfenahme eines teuren, opisch aktiven Trägermaterials, lediglich im analytischen Maßstab möglich ist.

Es ist die Aufgabe der vorliegenden Erfindung ein stereospezifisches Herstellungsverfahren zur Herstellung enantiomerenreiner Hetrazepine zur Verfügung zu stellen. Es ist ein weiteres Ziel dieser Erfindung, die so hergestellten enantiomerenreinen Verbindungen zur Herstellung von Arzneimitteln mit PAF-antagonistischer Wirkung zu verwenden.

Ausgehend von optisch aktiver Cyclopentanon-3-carbonsäure erfolgt die Herstellung der enantiomerenreinen Hetrazepine der allgemeinen Formel I in einer 11-stufigen Synthese, wie es im Reaktionsschema dargestellt ist. Die als Ausgangsverbindung eingesetzte "enantiomerenreine" Cyclopentanon-3-carbonsäure ist aus dem entsprechenden Racemat durch Racematspaltung mittels Brucin nach literaturbekannten Verfahren zugänglich. Während im allgemeinen bei stereospezifischen Synthesen -insbesondere bei vielstufigen Synthesen - sehr hohe Anforderungen an die optische Reinheit der Ausgangsverbindungen (in der Regel größer 99 % = ee 98 %) gestellt werden, ist es nach dem erfindungsgemäßen Verfahren

vollkommen ausreichend, wenn der Enantiomerenüberschuß einer Komponente 80 % beträgt.

Nachfolgend wird das erfindungsgemäße Verfahren anhand des konkret beschriebenen Reaktionsschemas näher erläutert. Verbindungen mit anderen - als den angegebenen - Resten lassen sich in Analogieverfahren herstellen.

Reaktionsschema

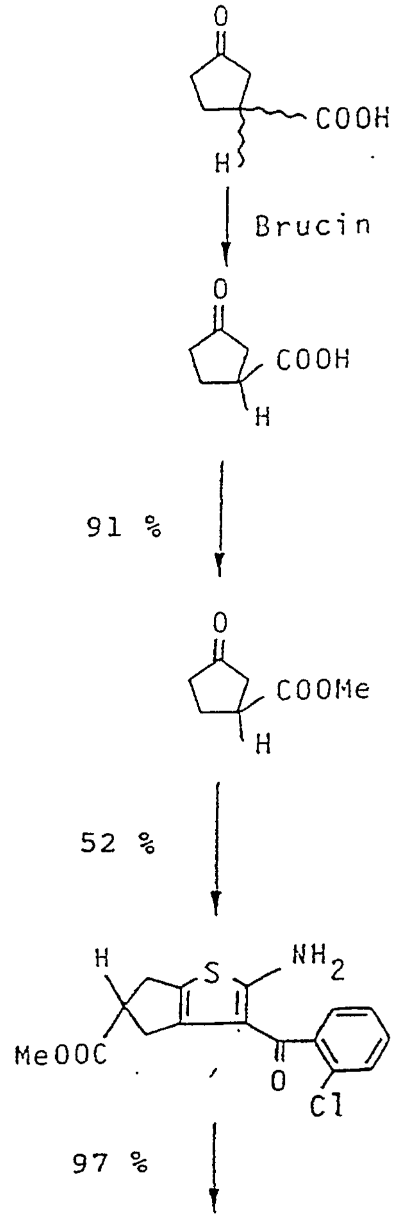


+ Säure (Ia)
$[\alpha]_D^{20}$ = +23.2°
(c=2, Methanol)

+ Ester (IIa)
$[\alpha]_D^{20}$ = +29.75°
(c=2, Methanol)

Thiophen (IIIa)
$[\alpha]_D^{20}$ = −97.9°
(c=0.58, Methanol)

Verbindung (IVa)
$[\alpha]_D^{20} = -43.3°$
(c=1, Methanol)

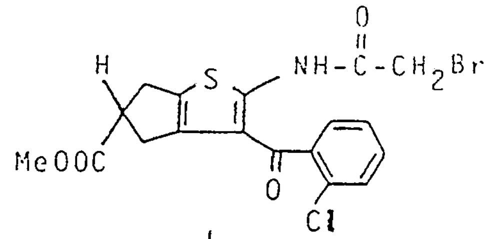

92 %

Verbindung (Va)
$[\alpha]_D^{20} = -45.2°$
(c=1, Methanol)

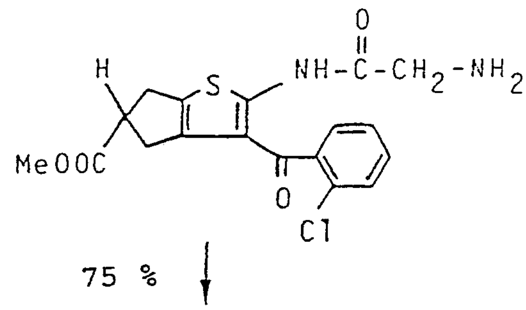

75 %

Verbindung (VIa)
$[\alpha]_D^{20} = -62.8°$
(c=0.42, Methanol)

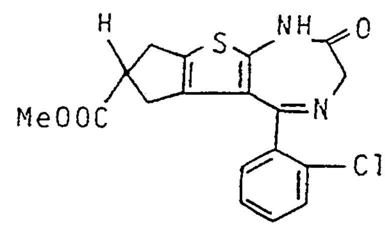

94 %

Verbindung (VIIa)
$[\alpha]_D^{20} = -55.1°$
(c=0.61, Methanol)

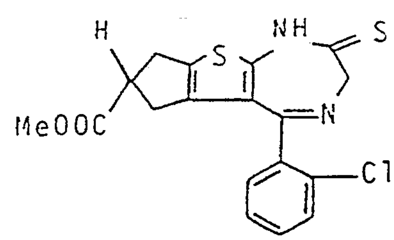

85 %

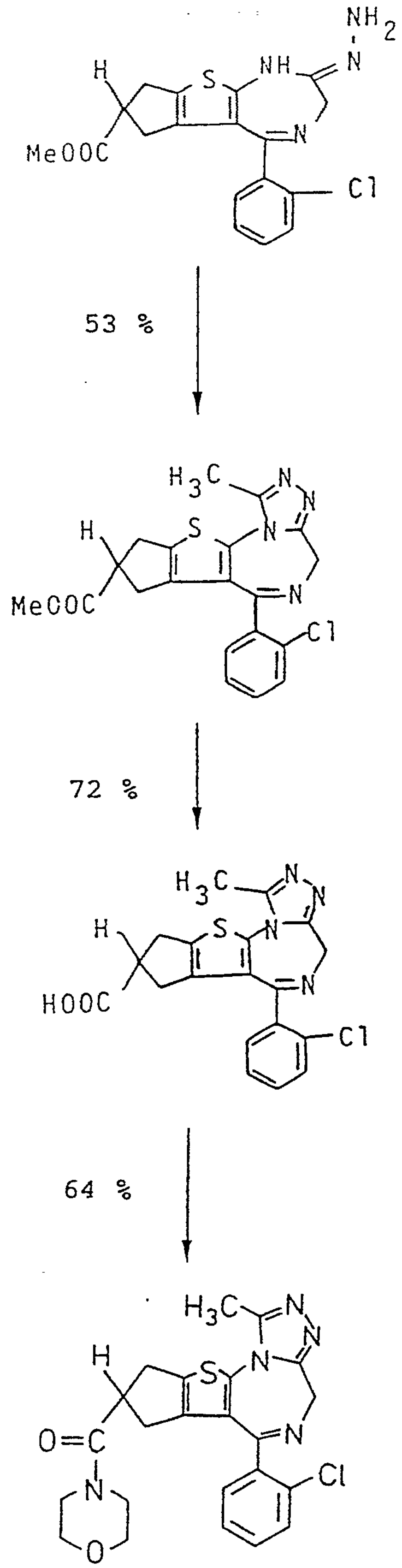


Verbindung (VIIIa)
$[\alpha]_D^{20}$ = -96.4°
(c=0.54, Methanol)

Verbindung (IXa)
$[\alpha]_D^{20}$ = -33.1°
(c=1, Methanol)

Verbindung (Xa)
$[\alpha]_D^{20}$ = -40.5°
(c=0.75, Methanol)

Verbindung (XIa)
$[\alpha]_D^{20}$ = - 12.2°
(c=0.32, Methanol)

Stufe I und II:

Ausgehend von der (+)-Säure mit einem Reinheitsgrad von 99,7 %, der erst nach der Brucinabtrennung durch erneute Umkristallisation erhalten wird, erfolgt die Überführung in den (+)-R-Cyclopentan-3-on-carbonsäuremethylester nach an sich bekannten Methoden, wobei die Enantiomerenreinheit des Produkts erhalten bleibt.

Stufe III:

Wird die Umsetzung des Esters mit o-Chlorcyanoacetophenon und Schwefel unter den in der europäischen Patentanmeldung 254 245 angegebenen Bedingungen in Dimethylformamid als Lösungsmittel durchgeführt, so ist zur Abtrennung von unerwünschten Nebenprodukten eine chromatographische Aufarbeitung erforderlich, die nicht zu einer Trennung der Enantiomeren führt. Zudem erfolgt unter der angewandten Reaktionsbedingung - auch beim Einsatz hochreiner Ausgangsprodukte (ee > 99 %) ein starker Abfall der optischen Aktivität auf einen Enantiomerenüberschuß von lediglich 60 %. Das dort offenbarte Verfahren ist somit für eine stereospezifische Synthese völlig ungeeignet.

Überraschenderweise wurde gefunden, daß bei einer Umsetzung in $C_1$-$C_4$ Alkoholen, bevorzugt in Methanol oder Ethanol als Lösungsmittel das gewünschte Enantiomere in hoher optischer Ausbeute in kristalliner Form erhalten werden kann.

Wird ein Ester mit einem Enantiomerenüberschuß (ee) von größer gleich 99 % eingesetzt, so erhält man das gewünschte Reaktionsprodukt direkt aus der Reaktionslösung in sehr hoher optischer Ausbeute (ee > 99 %) ohne weitere Umkristallisation. Als Carbonsäureester eignen sich prinzipiell alle Ester, soweit sie sich später verseifen lassen. Bevorzugt sind $C_1$-$C_4$ Alkylester - besonders bevorzugt finden die Methyl- und Ethylester Verwendung.

Aufgrund der Eigenschaft, daß das gewünschte Enantiomer in optisch reiner Form direkt aus dem Reaktionsgemisch auskristallisiert, werden an die eingesetzten Ausgangsverbindungen bezüglich ihrer optischen Reinheit nicht so hohe Anforderung gestellt. Wie in der nachfolgenden Tabelle I dargestellt, werden auch noch mit Cyclopentan-3-carbonsäuren gute Ergebnisse erhalten, die einen Enantiomerenüberschuß von 88 % aufweisen. Eine Trennung der Enantiomeren wird auf der Stufe der Thiophensynthese selbst dann noch erreicht, wenn der Enantiomerenüberschuß (e.e.) der eingesetzten Carbonsäure lediglich 60 % beträgt, wobei eine sinnvolle technische Nutzung des Verfahrens ab einem Enantiomerenüberschuß > 80 % möglich ist.

Tabelle I

| Stufe | Enantiomerenüberschuß e.e. [%] $[\alpha]_D^{20}$ -Werte | | | |
|---|---|---|---|---|
| I | 98 | 88 | 80 | 60 |
| III $K_1$<br>III $K_2$<br>III $K_3$ | - 93,5° | - 84°<br>- 80°<br>- 94,6° | - 80°<br>- 78°<br>- 89,2° | - 65°<br>- 58,2°<br>- 75,6° |
| $K_1$ = Rohprodukt aus Ethanol<br>$K_2$ = eingeengte Mutterlauge, Nachfällung<br>$K_3$ = $K_1$ einmal aus Ethanol umkristallisiert. | | | | |

Ist der Enantiomerenüberschuß des als Ausgangsmaterial eingesetzten Esters geringer, so sinkt selbstverständlich die optische Ausbeute des Reaktionsproduktes. Dieser Nachteil kann jedoch durch einfache einmalige Umkristallisation ausgeglichen werden.

Der dabei auftretende Ausbeuteverlust wird durch die weitaus geringeren Herstellungskosten eines nicht so hochgereinigten Ausgangsmaterials (Cyclopentan-3-carbonsäure) mehr als kompensiert.

Durch Aufarbeitung der Mutterlauge und der daraus gewonnenen Nachfällung kann die Ausbeute zusätzlich erhöht werden.

Geeignete Lösungsmittel zur Umkristallisation sind beispielsweise Acetonitril, Aceton, Methylethylketon, Essigester - bevorzugte Lösungsmittel sind $C_1$ bis $C_3$ Alkohole, besonders bevorzugt Methanol oder Ethanol. Es können selbstverständlich auch Lösungsmittelgemische verwendet werden. In der Tabelle II sind entsprechende Beispiele für eine Umkristallisation aufgeführt. Optisch reines Thiophenderivat der Stufe III mit $[\alpha]_D^{20}$ = -92.5° wurde im Verhältnis 8 : 1 mit dem anderen Enantiomeren gemischt, der daraus resultierende Drehtwert betrug $[\alpha]_D^{20}$ -69,8°. Die nach einmaliger Umkristallisation erhaltenen Drehwerte sind in der Tabelle aufgeführt.

Tabelle II

| | Drehwert $[\alpha]_D^{20}$ |
|---|---|
| Thiophenderivat (Stufe III) | - 92,5° |
| Enantiomerenverhältnis 8 : 1 | - 69,8° |
| Umkrst. aus Lsgm. | |
| $CH_3CN$ | - 76,8° |
| Aceton | - 83,6° |
| Methylethylketon | - 83,0° |
| Essigester | - 83,0° |
| Ethanol | - 80,0° |
| Methanol | - 81,2° |

Stufe IV bis X

Nach an sich bekannten Methoden erfolgt die weitere Umsetzung gemäß den im Verfahrensschema angegebenen Stufen IV bis X. Überraschenderweise erfolgt auch unter basischen Bedingungen, d.h. bei der Umsetzung mit Ammoniak und Hydrazin, keine Racemisierung. Die optisch aktiven Carbonsäurederivate der allgemeinen Struktur X sind wertvolle Zwischenprodukte zur Herstellung weiterer enantiomerenreiner Hetrazepine und werden als solche beansprucht.

Enantiomerenreine Verbindungen der allgemeinen Formel I worin X=CH und Y=Stickstoff bedeuten, können ausgehend von dem Thion der Stufe VII in Analogie zu dem in der europäischen Patentanmeldung 254 245 offenbarten Verfahren durch Umsetzung mit einem Aminoalkin oder einem α-Aminoaldehydalkylacetal erhalten werden. Aufgrund der bei der Cyclisierung angewandten sauren Reaktionsbedingungen tritt keine Racemisierung der Verbindungen ein.

Ausgehend von den optisch aktiven Carbonsäuren der allgemeinen Struktur X können die entsprechenden optisch aktiven Säureamide nach folgenden Verfahren hergestellt werden.

Die Umsetzung reaktiver Amine erfolgt bei Raumtemperatur in Gegenwart von Carbonyldiimidazol oder Dicyclohexylcarbodiimid in einem inerten organischen Lösungsmittel. Höhere Temperaturen sind wegen einer Racemisierung zu vermeiden.

Bei der Verwendung weniger reaktiver Amine ist es sinnvoll, zunächst die Carbonsäure in das entsprechende Carbonsäurechlorid zu überführen und anschließend mit dem Amin umzusetzen.

Nach den oben beschriebenen Verfahren können beispielsweise die enantiomerenreinen Carbonsäureamide der allgemeinen Formel I der europäischen Patentanmeldung 254 245 - auf die diesbezüglich hiermit inhaltlich Bezug genommen wird - vorteilhaft hergestellt werden.

In Abhängigkeit von der Chiralität der eingesetzten Cyclopentanon-3-carbonsäure können nach dem erfindungsgemäßen Verfahren sowohl R- wie auch S-konfigurierte (bzgl. d. 3-Position) Cyclopentathienotriazolodiazepine wie auch die entsprechenden Imidazo-Derivate in hoher optischer Reinheit (e.e. > 99 %) erhalten werden.

Die nachfolgenden Beispiele sollen die Erfindung erläutern, ohne sie auf die dort beschriebenen Reste einzuschränken.

## Experimentelle Beschreibung:

### Beispiel 1

#### a) (+)-R-Cyclopentan-3-on-carbonsäure (1a)

Die genannte optisch aktive Ausgangsverbindung kann z.B. durch Racematspaltung mit Brucin gemäß K. Toki, Bull. Chem. Soc. Japan 31, 333 (1958) bzw. M. Kinoshita und S. Umezawa, Bull. Chem. Soc. Japan 32, 223 (1959) erhalten werden.

(1a): $[\alpha]_D^{20}$ = + 33.2° (c=2, Methanol), ee = 99,4 %

Die quantitative Bestimmung der Enantiomerenreihheit erfolgt mittels HPLC nach Überführung von 1 in das 3,5-Dinitroanilid mittels 2-Ethoxy-1-ethoxycarbonyl-1,2-dihydrochinolin als Kopplungsreagens (stationäre Phase "D-Naphtylalanin", mobile Phase n-Hexan/Propanol-2/Acetonitril = 90:9:1).

#### b) (+)-R-Cyclopentan-3-on-carbonsäuremethylester (2a)

Eine Mischung aus 768,7 g 1a, 727 ml Methanol, 580 ml Tetrachlorkohlenstoff und 30 ml konzentrierter Schwefelsäure wird 4-6 Stunden zum Rückfluß erhitzt. Mittels Dünnschichtchromatographie überprüft man die Vollständigkeit der Umsetzung, verdünnt nach dem Abkühlen mit 2 1 Methylenchlorid, wäscht die organische Phase mit Wasser, 2%iger Bicarbonat-Lösung und anschließend nochmals mit Wasser, trocknet mit Natriumsulfat und zieht anschließend das Solvens vollständig ab.

Man erhält 2a in 91 % Ausbeute als Öl mit $[\alpha]_D^{20}$ = + 29,75° (c=2, Methanol). Der so erhaltene Ester wird ohne weitere Aufreinigung weiter umgesetzt.

#### c) (-)-S-2-Amino-3-(2-chlorbenzoyl)-5,6-dihydro-5-methoxycarbonyl-4H-cyclopenta[b]thiophen (3a)

Zu einer Suspension von 1,056 kg o-Chlorcyanoacetophenon und 188 g Schwefel in 4,18 1 Methanol werden bei 20 °C innerhalb von 10 Minuten 406 ml Triethylamin zugetropft. Nach weiteren 10 Minuten gibt man 836 g (+)-R-Cyclopentan-3-on-carbonsäuremethylester (2a) zu, erhitzt die Suspension 3 Stunden zum Rückfluß und saugt nach dem Erkalten das entstandene kristallisierte Produkt ab. Umkristallisation aus 35 1 Methanol ergibt reines 3a vom Fp. 176-178 °C in 52 % Ausbeute mit $[\alpha]_D^{20}$ = 97,9° (c=0.58, Methanol).

Eine analytische Überprüfung der Enantiomerenreinheit von 3a ist mittels HPLC möglich nach Überführung von 3a in das 5-Carboxylderivat durch saure Verseifung und anschließende weitere Umsetzung zum 5-Morpholinocarbonyl-Derivat (Stationäre Phase β-Cyclodextrin, chemisch an sphärisches Kieselgel, - Korngröße 5 μ - gebunden, mobile Phase Wasser/methanol = 85 : 15).

#### d) (-)-S-2-Bromacetylamino-3-(2-chlorbenzoyl)-5,6-dihydro-5-methoxycarbonyl-4H-cyclopenta[b]thiophen (4a)

Zu einem Gemisch von 8,2 1 Toluol, 685 ml Wasser, 126 g Natriumhydrogencarbonat und 335 g 3a werden bei Raumtemperatur 132 ml Bromacetylbromid zugetropft und anschließend 2 Stunden bei 60 °C gerührt. Nach dem Abkühlen und mehrmaligen Ausschütteln mit Wasser erhält man aus der organischen Phase einen orangefarbenen öligen Rückstand, der aus heißem Ethanol zur Kristallisation gebracht wird. Man erhält die Verbindung 4a in einer Ausbeute von 97 %, Fp. 117-119 °C, $[\alpha]_D^{20}$ = - 43,2° (c=1, Methanol).

#### e) (-)-S-2-Aminoacetylamino-3-(2-chlorbenzoyl)-5,6-dihydro-5-methoxycarbonyl-4H-cyclopenta[b]thiophen (5a)

In eine Lösung von 256 g 4a in 2,5 1 Essigester wird bei 20° 2 Stunden Ammoniak eingeleitet, 12 Stunden weiter gerührt, die organische Phase wird anschließend mehrmals mit Wasser gewaschen und nach dem Abziehen des Solvens der Rückstand ohne weitere Reinigung für die anschließende Ringschlußreaktion verwendet.

Zur Substanzcharakterisierung kann eine Probe des öligen Rohprodukts aus heißem Aceton kristallin mit einem Schmelzpunkt Fp=165 °C und $[\alpha]_D^{20}$ = -45,2° (c=1, Methanol) erhalten werden.

f) (-)-S-7-Carbomethoxy-5-(2-chlorphenyl)-1,2,7,8-tetrahydro-3H,7H-cyclopenta[4,5]thieno[2,3-e][1,4]diazepin-2-on (6a)

219,9 g 5a, 660 g Kieselgel und 3,5 1 Toluol werden 3 Stunden am Wasserabscheider zum Rückfluß erhitzt. Das Kieselgel wird abgesaugt und mehrmals mit insgesamt 3 1 Methanol heiß extrahiert. Nach dem Abziehen des Solvens und Umkristallisation des Rückstandes aus 200 ml Acetonitril erhält man in 73 % Ausbeute die Verbindung 6a als Kristalle von Fp. 96-98 °C und $[\alpha]_D^{20}$ = - 62,8° (c=0.42, Methanol).

g) (-)-S-7-Carbomethoxy-5-(2-chlorphenyl)-1,2,7,8-tetra-hydro-3H,7H-cyclopenta[4,5]thieno[2,3-e][1,4]-diazepin-2-thion (7a)

256 g 6a, 181,3 g Phosphorpentasulfid und 136,9 g Natriumhydrogencarbonat werden in 2 1 Diglyme 3 Stunden bei 70 °C gerührt und bei 70 °C mit 1,3 1 Ethanol und anschließend mit 5,1 1 Wasser versetzt. Nach dem Abkühlen werden die ausgefallenen Kristalle abgesaugt und bei 50 °C getrocknet. Fp. 140-145 °C, Ausbeute 94%, $[\alpha]_D^{20}$ = - 55,1° (c=0.61, Methanol).

h) (-)-S-7-Carbomethoxy-5-(2-chlorphenyl)-7,8-dihydro-2-hydrazino-3H,7H-cyclopenta[4,5]thieno[2,3-e][1,4]-diazepin (8a)

Zu 381 g 7a in 3,8 1 Tetrahydrofuran tropft man bei 20 °C 58 g Hydrazinhydrat zu, rührt die entstandene Suspension 2 Stunden, filtriert über Kieselgel, zieht das Solvens ab und verreibt den Rückstand mit 1 1 Isopropanol. Man erhält die Verbindung 8a nach dem Trocknen als Kristalle vom Fp. 184-186 °C, Ausbeute 85 %, $[\alpha]_D^{20}$ = - 96,4° (c=0.54, Methanol).

i) (-)-S-8-Carbomethoxy-6-(2-chlorphenyl)-8,9-dihydro-1-methylcyclopenta[4,5]thieno[3,2-f][1,2,4]triazolo-[4,3-a][1,4]diazepin (9a)

Eine Mischung aus 193 g 8a, 93 g Orthoessigsäuretriethylester und 3,8 1 Ethanol werden 1 Stunde bei 60 °C gerührt, anschließend über Kieselgur filtriert, das Solvens wird abgezogen und der Rückstand direkt für die Verseifung eingesetzt.

Zur Substanzcharakterisierung kann eine Probe des Rückstands an Kieselgel mit Methylenchlorid/Methanol = 9:1 chromatographiert werden. Man erhält 6a als amorphes Pulver, $[\alpha]_D^{20}$ = -33,1° (c=1, Methanol).

j) (-)-S-8-Carboxy-6-(2-chlorphenyl)-8,9-dihydro-1-methyl-4H,7H-cyclopenta[4,5]thieno[3,2-f][1,2,4]-triazolo-[4,3-a][1,4]diazepin (10a)

Ein Gemisch aus 280 g 9a, 0,5 1 Tetrahydrofuran und 2,8 1 2N Salzsäure wird 1 Stunde bei 60 °C gerührt, über Kieselgur filtriert, mit 1 1 Methylenchlorid versetzt und mit 25 %iger Natronlauge auf pH 7,5 eingestellt. Die organische Phase wird abetrennt und die wäßrige Phase mit Methylenchlorid gewaschen. Nach Zugabe von 0,9 1 Methylenchlorid und 0,1 1 Methanol wird die wäßrige Phase mit 32 % Salzsäure auf pH 4,5 eingestellt und der Ansatz extraktiv mit Methylenchorid/Methanol = 9 : 1 aufgearbeitet. Der Rückstand wird aus 0,5 1 Methanol umkristallisiert und mit 0,5 1 Ether versetzt. Man isoliert die Verbindung 10a in einer Ausbeute von 73 %, Fp. 215-220 ° C, $[\alpha]_D^{20}$ = -40,55° (c=0.75, Methanol).

k) (-)-S-6-(2-Chlorphenyl)-8,9-dihydro-1-methyl-8-(morpholinocarbonyl)-4H,7H-cyclopenta[4,5]thieno-[3,2-f]-[1,2,4]triazolo[4,3-a][1,4]diazepin (11a)

119 g der Verbindung 10a werden in 1,2 1 Methylenchlorid gelöst und mit 48,6 g Carbonyldiimidazol versetzt. Nach 30 Minuten Rühren werden 26,1g Morpholin hinzugegeben und 12 Stunden bei Raumtemperatur gerührt. Nach Zugabe von 0,5 1 Wasser wird der Ansatz mit Methylenchlorid extraktiv aufgearbeitet.

Der Rückstand wird mit 130 ml warmen Isopropanol versetzt und die Kristalle abgesaugt. Die Kristalle können nochmals aus Ethanol (1:3) umkristallisiert werden. Man isoliert die Verbindung 11a als Kristalle vom Fp. 212-214 °C, Ausbeute 81 %, $[\alpha]_D^{20}$ = -12,78° (c = 0.657, Methanol).

Die Bestimmung der Enantiomerenreinheit der Diazepine 9a, 10a und 11a erfolgt mittels HPLC (stationäre Phase Poly-N-acryloyl-L-phenylalaninethylester auf sphärischem Kieselgel, Korngröße 7 µm, mobile Phase Dioxan/n-Hexan = 1 : 1).

11a: Konfiguration: (-)-(S)ee> 99,6 %; <0,2 % (+)-R; gleiches Ergebnis gilt damit auch für 9a und 10a: ee> 99,6 %.

Beispiel 2

Ausgehend von (-)-S-Cyclopentan-3-on-carbonsäure (1b) mit $[\alpha]_D^{20}$ = -23,25° (c=2, Methanol) kann analog die entsprechende Enantiomerenreihe hergestellt werden:

| Ausbeute | Fp °C | $[\alpha]_D^{20}$ |
|---|---|---|
| (-)-(S)-2b 93 % | | - 33,1° (c=2) |
| (+)-(R)-3b 49 % | 176-177 | + 97,3° (c=0.77) |
| (+)-(R)-4b 95 % | 118-120 | + 43,4° (c=0.87) |
| (+)-(R)-5b | | + 48,1° (c=1) |
| (+)-(R)-6b 75 % | 95 | + 61,17° (c=1.76) |
| (+)-(R)-7b 90 % | -- | + 46,5° (c=0.587) |
| (+)-(R)-8b 90 % | amorph | + 48,2° (c=1) |
| (+)-(R)-9b 31 % | amorph | + 44,4° (c=0.25) |
| (+)-(R)-10b 50 % | amorph | + 24,4° (c=1) |
| (+)-(R)-11b 30 % | 215-217 | + 12,3° (c=1) |

Alle Drehwerte ($[\alpha]_D^{20}$) wurden in Methanol gemessen.

Beispiel 3

(-)-S-6-(2-Chlorphenyl)-8,9-dihydro-8-(N,N-dipropylaminocarbonyl)-1-methyl-4H,7H-cyclopenta[4,5]thieno[3,2-f]-[1,2,4]triazolo[4,3-a][1,4]diazepin

Zu 2,0 g 10a in 50 ml Methylenchlorid tropft man bei 20°C 0,6 g Thionylchlorid zu, erhitzt 2 Stunden zum Rückfluß und tropft anschließend bei 0°C Dipropylamin bis zum pH-Wert 5,6-6,2 zu. Nach weiteren 2 Stunden Rühren und Waschen mit Wasser wird die Methylenchloridphase über Kieselgel abfiltriert und der Rückstand an Kieselgel mit Methylenchlorid/Methanol = 98 : 2 als Eluens chromatographiert. Man erhält die gewünschte Verbindung in 62 % Ausbeute als amorphes Pulver, $[\alpha]_D^{20}$ = -11,7° (c=1, Methanol), ee >99,6 %.

Beispiel 4

Analog wird aus 10b das (+)-R-Enantiomere hergestellt $[\alpha]_D^{20}$ = +11,5° (c=1, Methanol); ee = 98,4 %.

**Ansprüche**

1) Verbessertes Verfahren zur Herstellung enantiomerenreiner Hetrazepine der allgemeinen Formel I

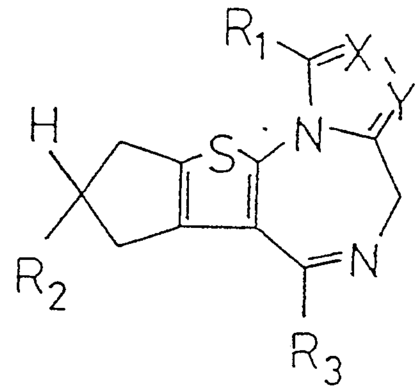

worin

$R_1$ Wasserstoff, eine verzweigte oder unverzweigte Alkylgruppe mit 1 bis 4 Kohlenstoffatomen, bevorzugt Methyl, die gegebenenfalls durch Hydroxy oder Halogen substituiert sein kann, eine Cyclopropylgruppe, eine Cyclobutylgruppe, eine Cyclopentylgruppe, Halogen, bevorzugt Chlor oder Brom;

$R_2$ COOR ($R = C_1$-$C_4$-Alkyl), COOH oder eine Säureamidgruppe;

$R_3$ Phenyl, wobei der Phenylring ein oder mehrfach, bevorzugt in 2-Stellung, durch Methyl, bevorzugt Halogen, Nitro und/oder Trifluormethyl substituiert sein kann;

$R_3$ Pyridyl, substituiertes Pyridyl, Thienyl oder substituiertes Thienyl, wobei der Heterocyclus bevorzugt über die 2-Stellung verknüpft ist;

und

X und Y beide Stickstoff oder X CH und Y Stickstoff bedeuten können, dadurch gekennzeichnet, daß man

a) einen optisch aktiven Cyclopentan-3-carbonsäureester in einem Enantiomerenüberschuß ee > 80 % mit einer Verbindung der allgemeinen Struktur

$N\equiv C\text{-}CH_2\text{-}CO\text{-}R_3$

in Gegenwart von Schwefel in einem $C_1$ bis $C_4$ Alkohol, bevorzugt in Methanol oder Ethanol umsetzt, die ausfallenden Kristalle isoliert, gegebenenfalls umkristallisiert,

b) anschließend

1) mit Bromacetylbromid,

2) mit Ammoniak,

3) unter Wasserabspaltung zu dem entsprechenden Diazepin-2-on cyclisiert,

c) anschließend mit einem Schwefelungsreagenz in das entsprechende Diazepinthion überführt;

d) dieses anschließend mit

al) Hydrazin und nachfolgende Umsetzung mit einem geeignetem Säurehalogenid oder einem geeignetem Orthoester

oder

a2) mit einem geeigneten Säurehydrazid der Formel $R_1\text{-}CO\text{-}NH\text{-}NH_2$

zu dem entsprechenden Triazolodiazepin der allgemeinen Formel I (X, Y beide N) umsetzt

oder

b1) mit einem Aminoalkin der Formel

$R - C\equiv C\text{-}CH_2\text{-}NH_2$

mit R = Wasserstoff oder

$C_1$-$C_3$- Alkyl

oder

b2) einem $\alpha$-Aminoaldehydalkylacetal der Formel

$NH_2\text{-}CH_2\text{-}CR_1\ (OR')_2$

mit $R' = C_1$-$C_4$-Alkyl

zu einem Imidazodiazepin der allgemeinen Formel I (X = CH, Y = N) umsetzt,

e) den so erhaltenen tetracyclischen Carbonsäureester (IX) in die Carbonsäure überführt

f) und gegebenenfalls nach Aktivierung der Carbonylfunktion mit einem Amin zum Säureamid umsetzt.

2) Verfahren zur Herstellung enantiomerenreiner (ee > 99 %) substituierter 2-Amino-3-benzoyl-5,6-dihydro-5-alkoxycarbonyl-4H-cyclopenta[b]thiophene durch Umsetzung der entsprechenden optisch aktiven Cyclopentan-3-carbonsäureester mit einen Enantiomerenüberschuß (ee) von mindestens 80 % mit den entsprechend phenylsubstituierten Cyanoacetophenonen in Gegenwart von Schwefel, dadurch gekennzeichnet, daß die Umsetzung in Methanol oder Ethanol als Lösungsmittel durchgeführt und gegebenenfalls einmal umkristallisiert wird.

3) Verfahren zur Herstellung enantiomerenreiner (ee > 99 %) substituierter 2-Amino-3-benzoyl-5,6-dihydro-5-methoxycarbonyl-4H-cyclopenta[b]-thiophene ausgehend von den entsprechenden optisch aktiven Thiophenen mit einem Enantiomerenüberschuß (ee) > 90 % dadurch gekennzeichnet, daß man aus einem Lösungsmittel oder Lösungsmittelgemisch ausgewählt aus der Gruppe Methanol, Ethanol, Isopropanol, Acetonitril, Aceton, Methylethylketon und Essigester umkristallisiert.

4) (-)-S-2-Amino-3-(2-chlorbenzoyl)-5,6-dihydro-5-methoxycarbonyl-4H-cyclopenta[b]thiophen

5) (+)-R-2-Amino-3-(2-chlorbenzoyl)-5,6-dihydro-5-methoxycarbonyl-4H-cyclopenta[b]thiophen

6) (-)-S-6-(2-Chlorphenyl)-8,9-dihydro-8-(N,N-dipropylaminocarbonyl)-1-methyl-4H,7H-cyclopenta[4,5]-thieno[3,2-f]-[1,2,4]triazolo[4,3-a][1,4]diazepin

7) (+)-R-6-(2-Chlorphenyl)-8,9-dihydro-8-(N,N-dipropylaminocarbonyl)-1-methyl-4H,7H-cyclopenta[4,5]-thieno[3,2-f]-[1,2,4]triazolo[4,3-a][1,4]diazepin

8) Verwendung einer nach Anspruch 1 hergestellten enantiomerenreinen Verbindung zur Herstellung von Arzneimittelzübereitungen.

Europäisches Patentamt

# EUROPÄISCHER RECHERCHENBERICHT

Nummer der Anmeldung

EP 90104890.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | DE - A1 - 3 724 031 (BOEHRINGER INGELHEIM KG) * Ansprüche 1,4,8-11 * ---- | 1-3,8 | C 07 D 495/14<br>A 61 K 31/55 |

RECHERCHIERTE SACHGEBIETE (Int Cl 5)

C 07 D 495/00

Der vorliegende Recherchenbericht wurde für alle Patentansprüche erstellt.

| Recherchenort | Abschlußdatum der Recherche | Prüfer |
|---|---|---|
| WIEN | 24-04-1990 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82